(19) Europäisches Patentamt
European Patent Office
Office européen des brevets

(11) Veröffentlichungsnummer: **0 334 281 A2**

# (12) EUROPÄISCHE PATENTANMELDUNG

(21) Anmeldenummer: **89105011.4**

(51) Int. Cl.4: **C07D 501/34 , A61K 31/545**

(22) Anmeldetag: **21.03.89**

Patentansprüche für folgende Vertragsstaaten: ES + GR

(30) Priorität: **22.03.88 DE 3809561**

(43) Veröffentlichungstag der Anmeldung: **27.09.89 Patentblatt 89/39**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Adam, Friedhelm, Dr.**
**Rheingaustrasse 46**
**D-6238 Hofheim am Taunus(DE)**
Erfinder: **Dürckheimer, Walter, Dr.**
**Im Lerchenfeld 45**
**D-6234 Hattersheim am Main(DE)**
Erfinder: **Fischer, Gerd, Dr.**
**Johannesallee 6**
**D-6230 Frankfurt am Main 80(DE)**
Erfinder: **Mencke, Burghard Dr.**
**Hauptstrasse 2**
**D-5409 Holzappel(DE)**
Erfinder: **Seliger, Hubert**
**Kallerstrasse 3**
**D-6000 Frankfurt am Main(DE)**
Erfinder: **Isert, Dieter, Dr.**
**Hamburger Strasse 1**
**D-6236 Eschborn(DE)**
Erfinder: **Klesel, Norbert, Dr.**
**Bornstrasse 50**
**D-6238 Hofheim am Taunus(DE)**
Erfinder: **Seibert, Gerhard, Prof. Dr.**
**Gläserweg 21**
**D-6100 Darmstadt(DE)**

## (54) Cephalosporinderivate und Verfahren zu ihrer Herstellung.

(57) Cephemcarbonsäureester der allgemeinen Formel

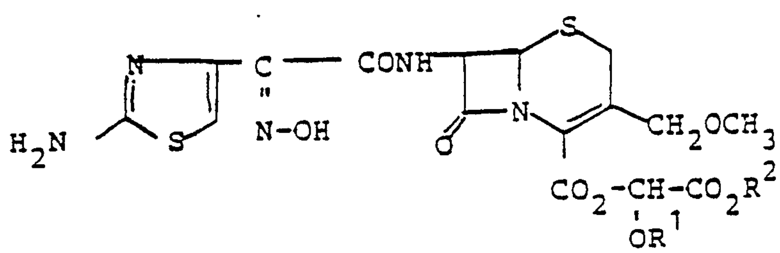

gegen bakterielle Infektionen wirksame pharmazeutische Präparate, die solche Cephemderivate enthalten, Verfahren zur Herstellung der Cephemderivate und der pharmazeutischen Präparate, sowie Verwendung der Cephemderivate zur Bekämpfung bakterieller Infektionen.

EP 0 334 281 A2

## Cephalosporinderivate und Verfahren zu ihrer Herstellung

Die Erfindung betrifft neue Cephalosporinderivate, die besonders für die orale Applikation geeignet sind, ein Verfahren zu ihrer Herstellung und pharmazeutische Zubereitungen, die solche Verbindungen enthalten.

Obwohl viele klinisch relevante Cephalosporine mit breitem antibakteriellem Spektrum entwickelt worden sind, eignen sich die meisten von ihnen nur für eine parenterale Verabreichung, da sie nach oraler Gabe, wenn überhaupt, nur sehr unzureichend resorbiert werden. In vielen Fällen ist es jedoch wünschenswert, dem Patienten hochwirksame Antibiotika in oraler Form zu geben.

Die bislang bekannten Cephalosporin-Antibiotika erfüllen nicht alle Anforderungen, die an ein solches Medikament gestellt werden müssen, nämlich eine hohe antibakterielle Aktivität gegen grampositive (speziell Staphylokokken) und gramnegative Erreger und gleichzeitig eine gute Resorption im Magen-Darm-Trakt.

In einigen Fällen ist es gelungen, die Resorption eines Cephalosporins im Gastrointestinaltrakt durch Veresterung der 4-Carboxylgruppe zu erhöhen. Da die Cephalosporinester in der Regel per se keine antibiotische Wirksamkeit aufweisen, muß die Esterkomponente so gewählt werden, daß der Ester nach der Resorption durch körpereigene Enzyme, wie Esterasen, wieder rasch und vollständig zum Cephalosporin mit freier Carboxylgruppe zurückgespalten wird.

Das Ausmaß der enteralen Resorption von Cephalosporinen ist maßgeblich abhängig von der chemischen Struktur des Cephalosporins und der jeweiligen Esterkomponente. Schon kleine strukturelle Variationen am Cephalosporin-Grundgerüst oder in der Esterkomponente können die Resorption beeinflussen. Das Auffinden geeigneter Komponenten ist rein empirisch.

So führt beispielsweise die Einführung eines sauren Substituenten in die 7$\beta$-Seitenkette von Aminothiazolyl-Cephalosporinen, wie z.B. im Cefixime, zu einer enteral resorbierbaren Verbindung, während Verbindungen mit neutralen Seitenketten, wie z.B. im Cefuroxim, nur in Form von Prodrug-Estern enteral resorbiert werden. Die Dosis-Wirkungs-Proportionalität ist dabei oft nicht linear und die erzielten therapeutischen Serumspiegel sind nicht befriedigend. Weitere Ester aus der Reihe der Aminothiazolyl-Cephalosporine werden beispielsweise in den EP-PSn 29 557, 34 536, 49 119 und 134 420 erwähnt.

Wir fanden nun durch systematisch durchgeführte in vivo-Untersuchungen in verschiedenen Tierspecies eine enge Gruppe von oral applizierbaren Ceph-3-em-4-carbonsäureestern, die eine ausreichende chemische Stabilität besitzen und durch ausgewogene Lipid- und Wasserlöslichkeit rasch und in therapeutisch beachtlichem Maße im Magen-Darm-Trakt resorbiert werden.

Gegenstand der Erfindung sind demnach Cephemcarbonsäureester der allgemeinen Formel I

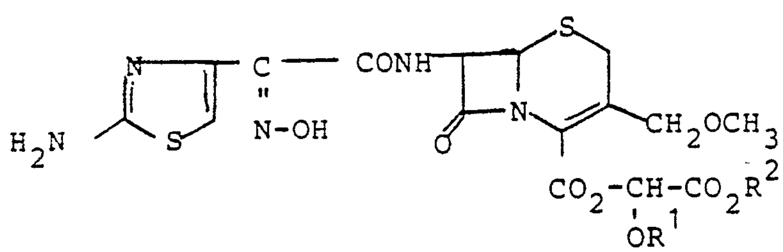

(I)

in der $R^1$ und $R^2$ die Bedeutung von $C_1$-$C_6$-Alkyl besitzen und die HO-Gruppe in syn-Position steht, sowie deren physiologisch verträgliche Säureadditionssalze.

Bevorzugte Bedeutungen für $R^1$ und $R^2$ sind

$R^1$ = $C_1$-$C_4$-Alkyl, wie z.B. Methyl, Ethyl, Propyl, Isopropyl, Butyl, sec.-Butyl, vorzugsweise Methyl oder Ethyl, insbesondere Ethyl,

$R^2$ = $C_1$-$C_4$-Alkyl, wie z.B. Methyl, Ethyl, Propyl, Isopropyl, Butyl, sec.-Butyl, vorzugsweise Methyl oder Ethyl, insbesondere Ethyl.

Als physiologisch verträgliche Säureadditionssalze kommen die für Cephalosporin-Antibiotika bekannten Salze in Betracht, wie z.B. das Hydrochlorid, Sulfat, Maleinat, Citrat, Acetat oder Formiat. Ihre Herstellung erfolgt in an sich bekannter Weise durch Zusammengeben der Komponenten in einem wäßrigen oder organischen Lösungsmittel oder einer geeigneten Lösungsmittelmischung.

Die Verbindungen der allgemeinen Formel I besitzen im Esterteil ein chirales Zentrum. Bei Verwendung racemischer Verbindungen der allgemeinen Formel III liegen die Cephemcarbonsäureester der allgemeinen Formel I als ein Gemisch von zwei Diastereomeren vor, die nach bekannten Methoden in die beiden

Einzelkomponenten trennbar sind.

Gegenstand der Erfindung ist weiterhin ein Verfahren zur Herstellung von Cephemcarbonsäureestern der allgemeinen Formel I

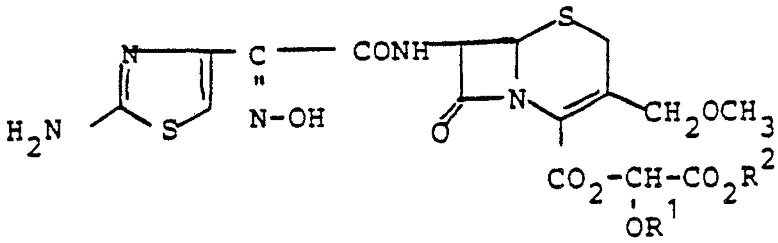

(I)

in der $R^1$ und $R^2$ die Bedeutung von $C_1$-$C_6$-Alkyl besitzen und die HO-Gruppe in syn-Position steht, sowie von deren physiologisch verträglichen Säureadditionssalzen, das dadurch gekennzeichnet ist, daß man

(a) eine Verbindung der allgemeinen Formel II

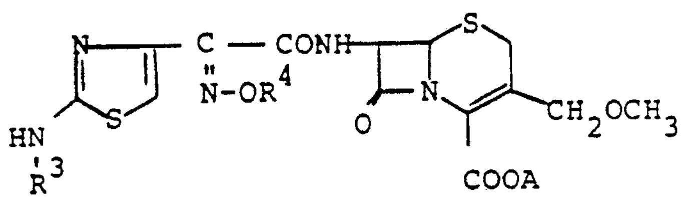

(II)

in der $R^3$ für eine Aminoschutzgruppe, $R^4$ eine leicht abspaltbare Gruppe und A ein Kation steht, mit einer Verbindung der allgemeinen Formel III

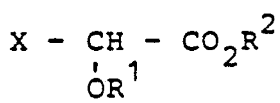

(III)

worin $R^1$ und $R^2$ die obige Bedeutung besitzen und X für eine Abgangsgruppe steht, zu dem Ester der allgemeinen Formel IV umsetzt

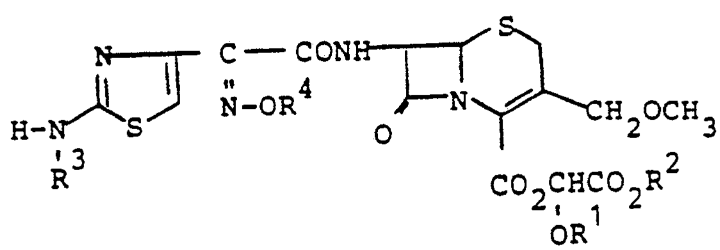

(IV)

und die Gruppen $R^3$ und $R^4$ in an sich bekannter Weise entfernt oder

(b) ein Verbindung der allgemeinen Formel V

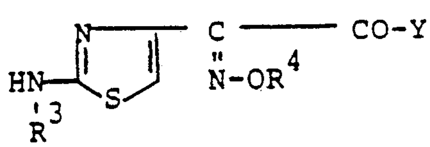

(V)

in der $R^3$ und $R^4$ die vorstehende Bedeutung besitzen und Y für eine aktivierende Gruppe steht, mit einer Verbindung der allgemeinen Formel VI

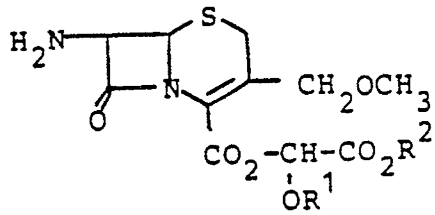

(VI)

in der $R^1$ und $R^2$ die vorstehende Bedeutung besitzen oder mit einem Salz dieser Verbindung, zu einer Verbindung der allgemeinen Formel IV umsetzt und die Gruppen $R^3$ und $R^4$ in an sich bekannter Weise abspaltet, oder

(c) eine Verbindung der allgemeinen Formel VII

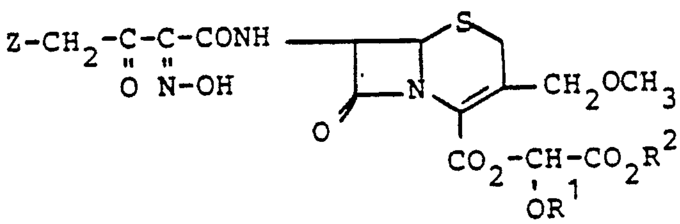

(VII)

in der Z für Halogen steht und $R^1$ und $R^2$ die vorstehende Bedeutung besitzen, mit Thioharnstoff zu Verbindungen der allgemeinen Formel I umsetzt und - falls erwünscht - die erhaltenen Verbindungen in ein physiologisch verträgliches Säureadditionssalz überführt.

In den allgemeinen Formeln II, IV und V steht $R^3$ für eine aus der Peptid- und Cephalosporinchemie bekannte Aminoschutzgruppe, vorzugsweise für Formyl, Chloracetyl, Bromacetyl, Trichloracetyl, Benzyloxycarbonyl, tert.-Butoxycarbonyl, Trityl oder Methoxytrityl und $R^4$ für eine ebenfalls aus der Peptid- und Cephalosporinchemie bekannte leicht abspaltbare Gruppe, vorzugsweise für Benzhydryl, Trityl, Tetrahydropyranyl oder 1-Methoxy-1-methylethyl. Besonders bevorzugt für $R^3$ ist Trityl und 1-Methoxy-1-methyl-ethyl.

In Formel III hat X die Bedeutung einer für Veresterungen allgemein bekannten Abgangsgruppe, wie beispielsweise Chlor, Brom, Jod, Phenylsulfonyloxy, p-Toluol-sulfonyloxy oder Methylsulfonyloxy, vorzugsweise Chlor, Brom oder Jod, insbesondere Jod.

Als Basen, die dem Kation A in der allgemeinen Formel II zugrundeliegen, seien z.B. genannt Natriumhydrogencarbonat, Kaliumhydrogencarbonat, Natriumcarbonat, Kaliumcarbonat, gegebenenfalls substituierte, alkylierte Aminbasen, wie z.B. Trimethylamin, Triethylamin, Diisopropylamin, Ethyldiisopropylamin, N,N-Dimethylanilin, N,N-Dimethylbenzylamin, 1,5-Diazabicyclo[4,3,0]non-5-en (DBN), 1,8-Diazabicyclo-[5,4,0]undec-7-en (DBU), Pyridin, Picolin oder 2,6-Dimethylpyridin. Bevorzugte Basen sind Natrium- oder Kaliumhydrogencarbonat, Natrium- oder Kaliumcarbonat, Triethylamin, N,N-Dimethylanilin, DBN oder DBU.

Durch Umsetzung der freien Carbonsäuren mit diesen Basen erhält man die Salze der allgemeinen Formel II, in der A für ein Kation, wie beispielsweise Natrium oder Kalium, aber auch für Magnesium oder Calcium oder für ein gegebenenfalls substituiertes alkyliertes Ammoniumion, wie beispielsweise Ammonium, Trimethylammonium, Triethylammonium, Tetrabutylammonium, Diisopropylammonium, Ethyldiisopropylammonium, Diazabicyclo[0,3,4]nonenium oder Diazabicyclo[0,4,5]undecenium steht. Bevorzugte Bedeutungen von A sind Natrium, Kalium, Triethylammonium, N,N-Dimethylanilinium, sowie das DBN- und DBU-ion.

In Verbindungen der Formel VII steht Z für ein Halogenatom, vorzugsweise für Brom oder Chlor.

Die Umsetzung der Verbindungen der Formel II mit den Verbindungen der Formel III kann in einem organischen Lösungsmittel bei etwa -20 bis etwa +50 °C, bevorzugt bei etwa 0 °C bis Raumtemperatur durchgeführt werden. Als Lösungsmittel können beispielsweise dienen Ketone, wie beispielsweise Aceton oder Methylethylketon, N,N-Dimethylformamid (DMF), N,N-Dimethylacetamid (DMA), N-Methylpyrrolidon oder Dimethylsulfoxid (DMSO). Bevorzugt sind DMF, DMA, N-Methylpyrrolidon und DMSO. Besonders bevorzugt ist DMF.

Die Abspaltung der Gruppen $R^4$ und $R^5$ aus den erhaltenen Verbindungen der Formel IV erfolgt in aus der Peptid- und Cephalosporinchemie an sich bekannter Weise, z.B. mit Trifluoressigsäure, verdünnter Salzsäure, vorzugsweise mit Ameisensäure unter Zugabe von etwas Wasser.

Setzt man eine Verbindung der Formel V mit einer Verbindung der Formel VI um, so stellt Y eine die

Carboxylgruppe aktivierende Gruppe dar, wie sie aus der Peptid- und Cephalosporinchemie für entsprechende Reaktionen bekannt ist, beispielsweise ein Halogenid, vorzugsweise Chlorid, eine aktivierende Estergruppe, beispielsweise mit 1-Hydroxybenzotriazol, N-Hydroxy-succinimid oder ein gemischtes Anhydrid, beispielsweise mit Benzolsulfonsäure oder Toluolsulfonsäure. Die Aktivierung der Carboxylgruppe ist auch in literaturbekannter Weise über die Zugabe eines Kondensationsmittels, wie z.B. eines Carbodiimids, möglich.

Die Verbindung der allgemeinen Formel VI läßt sich als solche oder in Form eines Salzes, beispielsweise des Tosylats, Hydrochlorids oder Hydrojodids einsetzen, wobei die Verwendung kristalliner Salze im Hinblick auf die Reinheit der Produkte vorteilhaft sein kann.

Die Umsetzung von Verbindungen der Formel V mit solchen der Formel VI kann in einem organischen Lösungsmittel, wie beispielsweise Methylenchlorid, Chloroform, Aceton, Methylethylketon, Dimethylformamid, Dimethylacetamid oder Wasser, oder auch in Mischungen dieser Lösungsmittel erfolgen.

Die Acylierungsreaktion kann zweckmäßigerweise bei Temperaturen von etwa -50 °C bis etwa +50 °C, vorzugsweise -40 °C bis +30 °C, gewünschtenfalls in Anwesenheit einer Base, wie beispielsweise Triethylamin oder Pyridin, durchgeführt werden. Der Basenzusatz dient dazu, die bei der Kondensation freiwerdende acide Komponente zu binden.

Der Ringschluß von Verbindungen der allgemeinen Formel VII mit Thioharnstoff kann nach an sich bekannten Verfahren, wie sie beispielsweise in der EP-PS 134 420 beschrieben sind, durchgeführt werden. Er gelingt z.B. glatt bei Temperaturen von etwa 0 bis 30 °C, vorzugsweise etwa 5 °C in organischen Lösungsmitteln, vorzugsweise aprotisch polaren Solventien, wie z.B. Dimethylformamid, Dimethylacetamid, Acetonitril oder Aceton.

Die Herstellung der Ausgangsverbindungen der allgemeinen Formel III erfolgt in literaturbekannter oder analoger Weise und ist in Chemische Ber. 44, S.3211, beschrieben, die Herstellung der Ausgangsverbindungen der allgemeinen Formel II in der EP-PS 34 536.

Die Herstellung von Ausgangsverbindungen der allgemeinen Formel V mit der aktivierten Carboxylgruppe erfolgt in literaturbekannter Weise, die zu den Verbindungen der Formel VI führende Veresterung auf dieselbe Weise, wie sie für die Herstellung der Ester der allgemeinen Formel IV beschrieben wurde.

Die Verbindungen der allgemeinen Formel VII lassen sich nach an sich bekannten Verfahren herstellen. So kann man beispielsweise (vgl. EP-PS 134 420) Diketen mit Brom, und das erhaltene Zwischenprodukt dann mit einer Verbindung der allgemeinen Formel VI umsetzen, wobei man ein Vorprodukt der Formel

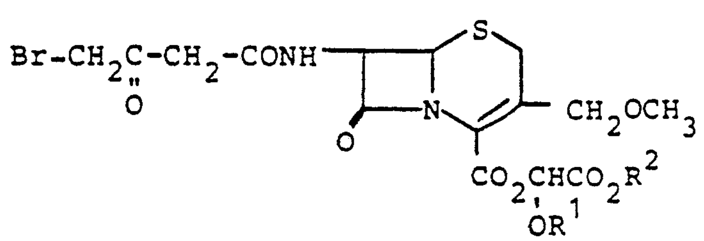

erhält, das anschließend durch Nitrosierung (vgl. ebenfalls EP-PS 134 420) in eine Verbindung der allgemeinen Formel VII überführt wird.

Die Ceph-3-em-4-carbonsäureester der allgemeinen Formel I besitzen eine Reihe physikochemischer und biologischer Eigenschaften, die sie zu wertvollen Cephalosporin-Antibiotika zur oralen Verabreichung machen. Sie sind stabile, farblose und in gängigen organischen Lösungsmitteln gut lösliche Verbindungen, die im Darm resorbiert, und nach Resorption aus dem Darm rasch zum antibiotisch aktiven Cephalosporin-Derivat der Formel

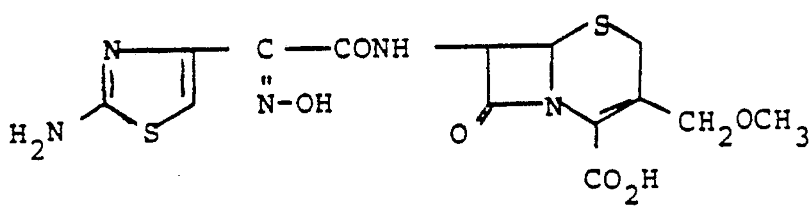

gespalten werden und sich daher hervorragend zur Behandlung von bakteriellen Infektionskrankheiten, wie z.B. der Infektion der Atemwege oder des Urogenitaltraktes eignen.

Die erfindungsgemäßen Verbindungen werden oral verabreicht in Form von üblichen pharmazeutischen Zubereitungen, wie z.B. Kapseln, Tabletten, Pulvern, Sirupen oder Suspensionen. Die Dosis hängt ab vom Alter, den Symptomen und dem Körpergewicht des Patienten sowie von der Dauer der Behandlung. Sie liegt jedoch in der Regel zwischen etwa 0,2 g und etwa 5 g täglich, vorzugsweise zwischen etwa 0,5 g und etwa 3 g täglich. Die Verbindungen werden vorzugsweise in aufgeteilten Dosen verabreicht, beispielsweise 2 bis 4-mal täglich, wobei die Einzeldosis beispielsweise zwischen 50 und 1000 mg Wirkstoff enthalten kann.

Die oralen Zubereitungen können die üblichen Trägerstoffe und/oder Verdünnungsmittel enthalten. So kommen beispielsweise für Kapseln oder Tabletten in Betracht Bindemittel, wie z.B. Gelatine, Sorbitol, Polyvinylpyrrolidon oder Carboxymethylcellulose, Verdünnungsmittel, wie z.B. Lactose, Zucker, Stärke, Calciumphosphate oder Polyethylenglykol, Gleitstoffe, wie z.B. Talkum oder Magnesiumstearat, für flüssige Zubereitungen z.B. wäßrige oder ölige Suspensionen, Sirupe oder ähnliche bekannte Zubereitungsformen.

Die folgenden Beispiele dienen zur weiteren Erläuterung der Erfindung, schränken sie jedoch nicht darauf ein. In den Beispielen ist in Übereinstimmung mit der Literatur die Angabe "Z" identisch mit "syn"-Position.

## A. Herstellung von Ausgangssubstanzen

### Herstellungsbeispiel 1

### Chlor-methoxy-essigsäuremethylester

21 g Phosphorpentachlorid (100 mmol) wurden mit 13,4 g (100 mmol) Dimethoxy-essigsäuremethylester übergossen und gerührt. Nach 4 Stunden ist das Phosphorpentachlorid unter leichtem Erwärmen vollständig in Lösung gegangen. Anschließend wurde langsam auf 140° C erhitzt und die Lösung noch 1 Stunde bei dieser Temperatur gehalten. Danach wurde der Rückstand im Vakuum fraktioniert destilliert.
Kp (22): 75 - 77° C; Ausbeute 9,5 g

### Herstellungsbeispiel 2

### Chlor-ethoxy-essigsäureethylester

21 g Phosphorpentachlorid (100 mmol) wurden mit 18 g (100 mmol) Diethoxy-essigsäureethylester übergossen und gerührt. Nach 4 Stunden ist das Phosphorpentachlorid unter leichtem Erwärmen vollständig in Lösung gegangen. Anschließend erhitzte man allmählich auf 140° C und hielt die Lösung bis zur Beendigung der Gasentwicklung (Ethylchlorid) bei dieser Temperatur. Danach wurde der Rückstand im Vakuum fraktioniert destilliert.
Kp (12): 80 - 81° C; Ausbeute 11,5 g

### Herstellungsbeispiel 3

### Chlor-n-propoxy-essigsäure-n-propylester

Stufe 1

Di-n-propoxy-essigsäure-n-propylester

Eine Lösung von 52,5 g (0,57 Mol) Glyoxylsäurehydrat und 338 g (1,78 Mol) Orthoameisensäure-tri-n-propylester, der 1,7 g p-Toluolsulfonsäure zugesetzt wurden, erhitzte man 30 Minuten unter Rückfluß und

destillierte anschließend 197 g eines Gemisches aus n-Propanol und Ameisensäure-n-propylester ab. Der Rückstand wurde über einer Kolonne destilliert.
Ausbeute: 76 g (62 %) ($Kp_{21}$ = 126 - 128° C).

Stufe 2

Chlor-n-propoxy-essigsäure-n-propylester

76 g (0,35 Mol) Di-n-propoxy-essigsäure-n-propylester wurden auf 72,5 g (0,35 Mol) Phosphorpentachlorid getropft und langsam auf 60° C erhitzt. Es trat Lösung ein. Nach 30-minütigem Erhitzen unter Rückfluß wurde im Vakuum fraktioniert destilliert.
Ausbeute: 59,4 g (88 %) ($Kp_{23}$ = 115 - 117° C)

| | | | | | |
|---|---|---|---|---|---|
| $C_8H_{15}C1O_3$ | (194,7) | Ber.<br>Gef. | C 49,35<br>C 49,6 | H 7,8<br>H 7,9 | Cl 18,25<br>Cl 17,8 |

**Herstellungsbeispiel 4**

**Chlor-n-butoxy-essigsäure-n-butylester**

Stufe 1

Di-n-butoxy-essigsäure-n-butylester

61 g (0,66 Mol) Glyoxylsäurehydrat wurden in 480 g (1,07 Mol) Orthoameisensäure-tri-n-butylester gelöst, 2 g p-Toluolsulfonsäure zugesetzt und 30 Minuten unter Rückfluß erhitzt. Eine anschließende fraktionierte Destillation lieferte die gewünschte Titelverbindung.
Ausbeute: 70,3 g (41 %) ($Kp_{0,09}$ = 90 - 92° C)

Stufe 2

Chlor-n-butoxy-essigsäure-n-butylester

70,3 g (0,27 Mol) Di-n-butoxy-essigsäure-n-butylester wurden auf 56,1 g (0,27 Mol) Phosphorpentachlorid getropft und unter Rühren langsam erhitzt. Nach 30 Minuten Erhitzen unter Rückfluß wurde im Vakuum fraktioniert destilliert. Man erhält so die gewünschte Titelverbindung.
Ausbeute: 46,2 g (77 %) ($Kp_{24}$ = 145 - 147° C)

| | | | | | |
|---|---|---|---|---|---|
| $C_{10}H_{19}C1O_3$ | (222,7) | Ber.<br>Gef. | C 53,9<br>C 54,2 | H 8,6<br>H 8,7 | Cl 15,95<br>Cl 15,3 |

**Herstellungsbeispiel 5**

7-[2-(2-Tritylaminothiazol-4-yl)-2-(Z)-trityl-oximinoacetamido]-3-(methoxymethyl)-3-cephem-4-carbonsäure

Stufe 1

2-(2-Tritylaminothiazol-4-yl)-2-(Z)-trityloximino-essigsäurechlorid

46,5 g (60 mmol) 2-(2-Tritylaminothiazol-4-yl)-2-(Z)-trityloximino-essigsäure-triethylammoniumsalz werden in 400 ml wasserfreiem Methylenchlorid gelöst und auf -70 °C abgekühlt. Dazu tropfte man eine Lösung von 12,3 g (60 mmol) Phosphorpentachlorid in 200 ml wasserfreiem Methylenchlorid so zu, daß die Temperatur nicht über -60 °C steigt. Danach rührte man noch 1 Stunde bei dieser Temperatur nach und engte danach die Lösung umgehend im Vakuum ein. Es wurde noch zweimal mit je 100 ml Methylenchlorid versetzt und einrotiert. Die Titelverbindung wurde ohne weitere Reinigung für die 2. Stufe eingesetzt.

Stufe 2

7-[2-(2-Tritylaminothiazol-4-yl)-2-(Z)-trityl-oximinoacetamido]-3-(methoxymethyl)-3-cephem-4-carbonsäure

Eine Lösung des Produktes aus Stufe 1 in 50 ml Methylenchlorid und 400 ml Aceton wurde zu einer auf 0 °C gekühlten Lösung von 18 g 7-Amino-3-methoxymethyl-ceph-3-em-4-carbonsäure in 400 ml Aceton/Wasser (1:1), der zuvor 5,6 g (66 mmol) Natriumhydrogencarbonat und 18 ml (132 mmol) Triethylamin zugesetzt wurden, getropft. Nach 2 Stunden Rühren bei 0 °C wurde mit 1N Salzsäure auf pH 4 gestellt und mit der doppelten Menge Essigester extrahiert. Anschließend wurden die Phasen getrennt und die organische Lösung mit 5 %iger Kaliumhydrogensulfat-Lösung extrahiert. Nach Trocknen über Magnesiumsulfat wurde eingeengt, der Rückstand in 150 ml Methylenchlorid gelöst und unter kräftigem Rühren in 1,8 l Diisopropylether eingetropft. Nach Absaugen erhielt man 45,7 g (85 %) der Titelverbindung.

**B. Ausführungsbeispiele**

**Beispiel 1**

**7-[2-(2-Aminothiazol-4-yl)-2-(Z)-hydroximinoacetamido]-3-(methoxy-methyl)-3-cephem-4-carbonsäure-(methoxy-methoxycarbonyl-methyl)-ester**

Zu einer Lösung von 6 g (6.7 mmol) 7-[2-(2-Tritylaminothiazol-4-yl)-2-(Z)-trityl-oximacetamido]-3-(methoxymethyl)-3-cephem-4-carbonsäure in 150 ml wasserfreiem Dimethylformamid wurden 460 mg (3.3 mmol) Kaliumcarbonat gegeben und 1 Stunde bei Raumtemperatur gerührt. Danach kühlte man auf 0 °C ab und versetzte mit 1 g (7.5 mmol) Chlor-methoxy-essigsäure-methylester und rührte 3 Stunden nach. Danach wurde das Lösungsmittel im Vakuum abgezogen und zwischen Essigester und Wasser verteilt. Die organische Phase wurde über Magnesiumsulfat getrocknet. Man erhielt 7 g Rohprodukt, das durch Flash-Chromatographie ($SiO_2$; Toluol/Essigester: 4 + 1) gereinigt wurde. Man erhielt 3,1 g eines Gemisches der beiden Diastereomeren.

3 g des Diastereomerengemisches wurden in 50 ml Ameisensäure gelöst und mit 10 ml Wasser versetzt. Nach 1 Stunde wurde das ausgefallene Triphenylcarbinol abgesaugt, das Filtrat eingeengt, der Rückstand mit Toluol aufgenommen und erneut das Lösungsmittel abgezogen. Das Produkt wurde anschließend mit Diisopropylether ausgerührt.

Ausbeute: 1,15 g der Titelverbindung als Gemisch der beiden Diastereomeren.

$^1$H-NMR (270 MHz, DMSO-$d_6$): $\delta$ = 3.23 (3H, s, $OCH_3$), 3.57 (2H, m, $SCH_2$), 3.52 und 3.55 (3H, 2 x s, $CHOCH_3$), 4.18 und 4.22 (2H, 2 x s, $CH_2OCH_3$), 5.23 (1H, dd, 6-H), 5.85 (1H, m, 7-H), 6.05 und 6.13 (1H, 2 x s, CH-$OCH_3$), 6.65 (1H, s, Thiazol-H), 7.1 (2H, breites s, $NH_2$), 9.45 (1H, d, NH), 11.3 (1H, s, Oxim-H).

**Beispiel 2**

**7-[2-(2-Aminothiazol-4-yl)-2-(Z)-hydroxyiminoacetamido]-3-(methoxy-methyl)-3-cephem-4-carbonsäure-(ethoxy-ethoxycarbonyl-methyl)-ester**

Zu einer Lösung von 6 g (6.7 mmol) 7-[2-(2-Tritylaminothiazol-4-yl)-2-(Z)-trityl-oximinoacetamido]-3-(methoxymethyl)-3-cephem-4-carbonsäure in 150 ml wasserfreiem Dimethylformamid wurden 536 mg (3,9 mmol) Kaliumcarbonat gegeben und 1 Stunde bei Raumtemperatur gerührt. Danach kühlte man auf 0° C ab und versetzte mit 1,3 g (8 mmol) Chlor-ethoxy-essigsäure-ethylester und rührte 4 Stunden nach. Nach Abzug des Lösungsmittels im Vakuum wurden zwischen Essigester und Wasser verteilt und die organische Phase über Magnesiumsulfat getrocknet. Man erhielt 8,2 g Rohprodukt, das durch Flash-Chromatographie ($SiO_2$; Toluol/Essigester: 4 + 1) gereinigt wurde. 3,75 g des so gewonnenen Diastereomerengemisches wurden in 56 ml Ameisensäure gelöst und mit 14 ml Wasser versetzt. Das kurz darauf ausfallende Triphenylcarbinol wurde nach 45 Minuten abgesaugt, das Filtrat eingeengt, der Rückstand mit Toluol aufgenommen und erneut das Lösungsmittel abgezogen.
Ausbeute: 1,4 g der Titelverbindung als Gemisch der beiden Diastereomeren.
$^1$H-NMR (270 MHz, DMSO-$d_6$): $\delta$ = 1.12 - 1.28 (6H, m, 2 x $CH_3$), 3.23 (3H, s, $OCH_3$), 3.47 (2H, m, $SCH_2$), 3.65 - 3.92 (2H, m, $CO_2CH_2CH_3$), 4.2 (4H, m, $CH_2OCH_3$ und $OCH_2CH_3$), 5.23 (1H, dd, 6-H), 5.85 (1H, m, 7-H), 6.03 und 6.17 (1H, s, CH-OEt), 6.65 (1H, s, Thiazol-H), 7.1 (2H, breites s, $NH_2$), 9.47 (1H, d, NH), 11.3 (1H, 2, Oxim-H)

## Beispiel 3

**7-[2-(2-Aminothiazol-4-yl)-2-(Z)-hydroiminoacetamido]-3-(methoxymethyl)-3-cephem-4-carbonsäure-(propoxy-propoxycarbonyl-methyl)-ester**

Zu einer Lösung von 12 g (13.4 mmol) 7-[2-(2-Tritylaminothiazol-4-yl)-2-(Z)-trityl-oximinoacetamido]-3-(methoxymethyl)-3-cephem-4-carbonsäure in 300 ml wasserfreiem Dimethylformamid wurden 920 mg (6.6 mmol) Kaliumcarbonat gegeben und 1 Stunde bei Raumtemperatur gerührt. Danach kühlte man auf 0° C ab, versetzte mit 3,1 g (16 mmol) Chlor-n-propoxy-essigsäure-n-propylester und rührte 3 Stunden nach. Nach Abzug des Lösungsmittels im Vakuum wurde zwischen Essigester und Wasser verteilt und die organische Phase über Magnesiumsulfat getrocknet. Man erhielt 14,8 g Rohprodukt, das nach Flash-Chromatographie ($SiO_2$; Toluol/Essigester: 4 + 1), 5,6 g Ester, als ein Gemisch von Diastereomeren, lieferte. Durch erneute Chromatographie (Toluol/Essigester: 3 + 1) erhielt man 3,8 g eines Gemisches der beiden Diastereomeren. Das Produkt löst man in 50 ml Ameisensäure, versetzte mit 12,5 ml Wasser und rührte 1 Stunde bei Raumtemperatur. Danach saugte man von entstandenem Triphenylcarbinol ab, nahm den Rückstand in Toluol auf, destillierte erneut das Lösungsmittel ab und verrührte mit Diisopropylether. Man erhielt 1,5 g der gewünschten Titelverbindung als ein Gemisch der beiden Diastereomeren.
$^1$H-NMR (270 MHz, DMSO-$d_6$): $\delta$ = 0.87 (6H, t, 2 x $CH_3$), 1.6 (4H, m, $OCH_2CH_3$), 3.22 (3H, s, $OCH_3$), 3.48 - 3.82 (4H, m, $CO_2CH_2$- und $SCH_2$), 4.15 (4H, m, $CH_2OCH_3$ und $CHOCH_2$-), 5.25 (1H, d, 6-H), 5.85 (1H, dd, 7-H), 6.05 (1H, s, CH-OProp.), 6.65 (1H, breites s, Thiazol-H), 7.1 (2H, breites s, $NH_2$), 9.45 (1H, d, NH), 11.25 (1H, breites s, Oxim-H).

## Beispiel 4

**7-[2-(2-Aminothiazol-4-yl)-2-(Z)-hydroximinacetamido]-3-(methoxymethyl)-3-cephem-4-carbonsäure-(butoxy-butoxycarbonyl-methyl)-ester**

Zu einer Lösung von 6 g (6.7 mmol) 7-[2-(2-Tritylaminothiazol-4-yl)-2-(Z)-trityl-oximacetamido-3-(methoxymethyl)-3-cephem-4-carbonsäure in 150 ml wasserfreiem Dimethylformamid wurden 460 mg (3.3 mmol) Kaliumcarbonat gegeben und 1 Stunde bei Raumtemperatur gerührt. Danach kühlte man im Eisabd und gab 1,8 g (8 mmol) Chlor-n-butoxyessigsäure-n-butylester zu. Nach 3,5 Stunden bei 90° C wurde das Lösungsmittel im Vakuum abgezogen und der Rückstand in Essigester aufgenommen und mit Wasser gewaschen. Nach Trocknen der organischen Phase über Magnesiumsulfat und Einengen erhält man 10,1 g eines öligen Rückstandes, der durch Flash-Chromatographie ($SiO_2$; Toluol/Essigester: 8 + 1) aufgetrennt wurde. Man erhielt 3,2 g eines amorphen Produktes, das in 50 ml Ameisensäure gelöst und sodann mit

12,5 ml Wasser versetzt wurde. Nach 2 Stunden wurde abgesaugt, eingeengt und zweimal in Toluol aufgenommen und erneut destilliert. Den Rückstand verrieb man mit Diisopropylether und saugte ab. Man erhielt 1,3 g der Titelverbindung als amorphes Pulver und Gemisch der beiden Diastereomeren.
$^1$H-NMR (270 MHz, DMSO-$d_6$): δ = 0.9 (6H, m, 2 x $CH_3$), 1.32 (4H, m, $CH_2CH_3$), 1.55 (4H, m, $CH_2$-$CH_2$-$CH_3$), 3.2 (3H, s, $OCH_3$), 3.45 - 3.85 (4H, m, $SCH_2$ und $CO_2CH_2$-), 4.17 (4H, m, $CH_2OCH_3$ und CH-O-$CH_2$-), 5.25 (1H, dd, 6-H), 5.75 (1H, m, 7-H), 6.03 und 6.15 (1H, 2 x s, CH-$OCH_2$), 6.15 (1H, s, Thiazol-H), 7.1 (2H, breites s, $NH_2$), 9.55 (1H, d, NH), 11.25 (1H, breites s, Oxim-H).

**Beispiel 5**

**7-[2-(2-Aminothiazol-4-yl)-2-(Z)-hydroxyimino-acetamido]-3-(methoxymethyl)-3-cephem-4-carbonsäure-(methoxy-methoxycarbonyl-methyl)-ester**

Vorstufe

2-(2-Tritylaminothiazol-4-yl)-2-(Z)-(1-methyl-1-methoxy)-ethoxy-imino-essigsäure-p-toluolsulfonsäureanhydrid

Zu einer Suspension von 6 g (10 mmol) 2-(2-Tritylaminothiazol-4-yl)-2-(Z)-(1-methyl-1-methoxy)-ethoxyimino-essigsäure-triethylammoniumsalz in 30 ml Aceton wurden 2,1 g (11 mmol) p-Toluolsulfonsäurechlorid gegeben und 1,5 Stunden bei Raumtemperatur gerührt. Anschließend versetzte man mit 40 ml Diethylether, kühlte auf -10 °C und saugte danach ab. Das Produkt wurde noch dreimal mit je 20 ml Ether nachgewaschen und getrocknet. Man erhielt 10 g Produkt, das aus einem Gemisch der Titelverbindung und Triethylamin-hydrochlorid bestand.

Stufe 1

7-[2-(2-Tritylaminothiazol-4-yl)-2-(Z)-(1-methyl-1-methoxy)-ethoxyiminoacetamido]-3-(methoxymethyl)-3-cephem-4-carbonsäure-(methoxy-methoxycarbonyl-methyl)-ester

0,73 g (3 mmol) 7-Amino-3-methoxymethyl-ceph-3-em-4-carbonsäure wurden in 30 ml Methylenchlorid suspendiert und mit 0,37 ml (2,4 mmol) 1,8-Diazabicyclo-(5,4,0)-undec-7-en (DBU) bei 0 °C versetzt. Die entstandene Lösung wurde noch 30 Minuten nachgerührt, anschließend 0.4 g (3 mmol) Chlormethoxy-essigsäuremethylester (Herstellungbeispiel 1) zugetropft und 30 Minuten bei dieser Temperatur nachgerührt. Danach gab man 2,8 g (3 mmol) des in der Vorstufe erhaltenen gemischten Anhydrids zu und hielt die Lösung 30 Minuten bei 0 °C.

Nach beendeter Reaktion wurde das Lösungsmittel im Vakuum abgezogen und der Rückstand chromatographiert ($SiO_2$; Toluol/Essigester = 1 + 1). Man erhielt 1,6 g (64 %) der gewünschten Verbindung als ein Gemisch der beiden Diastereomeren.
IR (KBr): 3060 und 3020 (w, Aromat), 1790 (s, β-Laktam), 1745 (s, Estercarbonyl), 1590 (s, Aromat), 700 (s, Aromat) $cm^{-1}$.

Stufe 2

7-[2-(2-Aminothiazol-4-yl)-2-(Z)-hydroxyimino-acetamido]-3-(methoxymethyl)-3-cephem-4-carbonsäure-(methoxy-methoxycarbonyl-methyl)-ester

1,5 g (1.8 mmol) der in Stufe 1 erhaltenen Verbindung wurden in 25 ml Ameisensäure gelöst und anschließend mit 5 ml Wasser versetzt. Nach 1 Stunde bei 0 °C wurde vom entstandenen Triphenylcarbinol abgesaugt und das Filtrat im Vakuum eingeengt. Den Rückstand nahm man in 100 ml Toluol auf und zog das Lösungsmittel erneut im Vakuum ab. Der Rückstand wurde mit Diisopropylether verrieben und so 700

mg (77 %) der gewünschten Titelverbindung als ein Gemisch der beiden Diastereomeren erhalten, die in allen Eigenschaften mit dem nach Beispiel 1 hergestellten Produkt identisch ist.

## Ansprüche

1. Cephemcarbonsäureester der allgemeinen Formel I

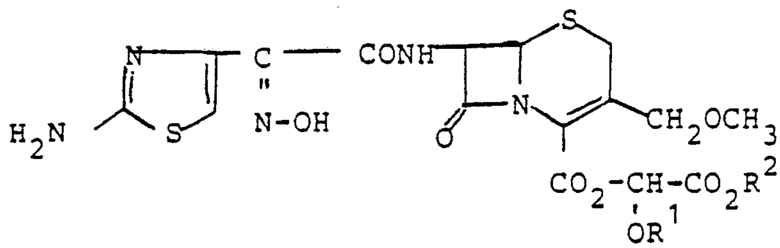

(I)

in der $R^1$ und $R^2$ die Bedeutung von $C_1$-$C_6$-Alkyl besitzen und die HO-Gruppe in syn-Position steht, sowie deren physiologisch verträgliche Säureadditionssalze.

2. Cephemcarbonsäureester der allgemeinen Formel I gemäß Anspruch 1, in der $R^1$ und $R^2$ für $C_1$-$C_4$-Alkyl stehen.

3. 7-[2-(2-Aminothiazol-4-yl)-2-(Z)-hydroxyimino-acetamido]-3-(methoxymethyl)-3-cephem-4-carbonsäure-(methoxy-methoxycarbonyl-methyl)-ester.

4. 7-[2-(2-Aminothiazol-4-yl)-2-(Z)-hydroxyimino-acetamido]-3-(methoxymethyl)-3-cephem-4-carbonsäure-(ethoxy-ethoxycarbonyl-methyl)-ester.

5. Verfahren zur Herstellung von Cephemcarbonsäureestern der allgemeinen Formel I und deren physiologisch verträglichen Säureadditionssalzen,

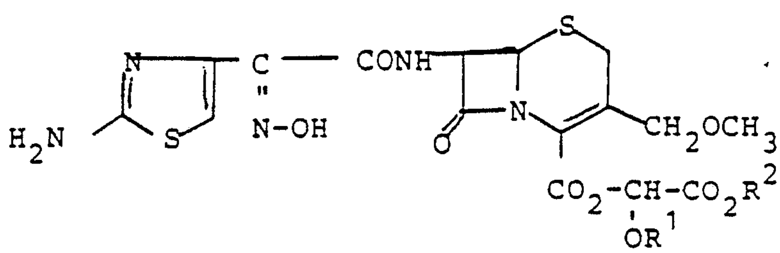

(I)

in der $R^1$ und $R^2$ $C_1$-$C_6$-Alkyl bedeuten und die HO-Gruppe in syn-Position steht, dadurch gekennzeichnet, daß man eine Verbindung der Formel II

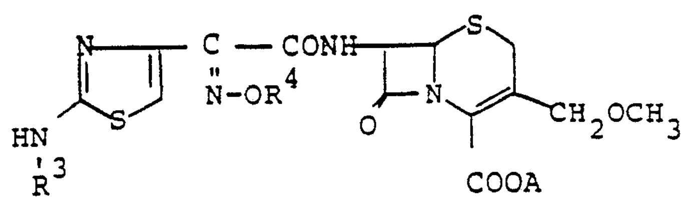

(II)

in der $R^3$ für eine Aminoschutzgruppe, $R^4$ eine leicht abspaltbare Gruppe und A ein Kation steht, mit einer Verbindung der allgemeinen Formel III

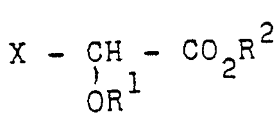

(III)

worin $R^1$ und $R^2$ die obige Bedeutung besitzen und X für eine Abgangsgruppe steht, zu dem Ester der allgemeinen Formel IV umsetzt

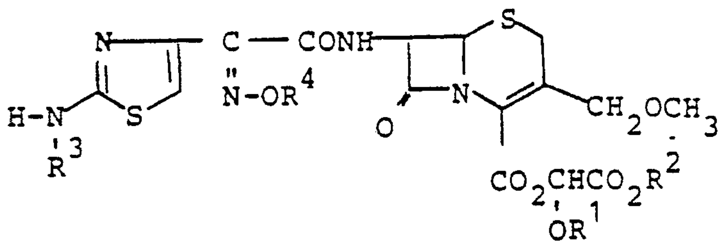

(IV)

und die Gruppen $R^3$ und $R^4$ in an sich bekannter Weise entfernt oder
eine Verbindung der allgemeinen Formel V

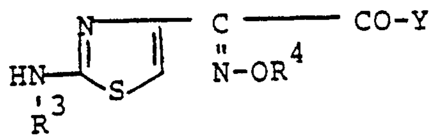

(V)

in der $R^3$ und $R^4$ die vorstehende Bedeutung besitzen und Y für eine aktivierende Gruppe steht, mit einer Verbindung der allgemeinen Formel VI

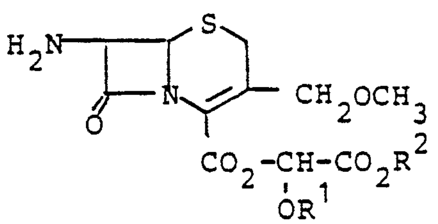

(VI)

in der $R^1$ und $R^2$ die vorstehende Bedeutung besitzen, oder mit einem Salz dieser Verbindung, zu einer Verbindung der allgemeinen Formel IV umsetzt und die Gruppen $R^3$ und $R^4$ in an sich bekannter Weise abspaltet, oder
eine Verbindung der allgemeinen Formel VII

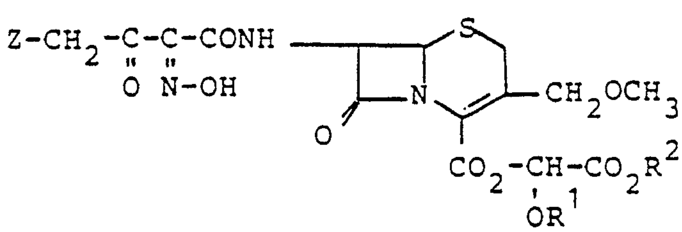

(VII)

in der Z für Halogen steht und $R^1$ und $R^2$ die vorstehende Bedeutung besitzen, mit Thioharnstoff zu Verbindungen der Formel I umsetzt und
- falls erwünscht - die erhaltenen Verbindungen in ein physiologisch verträgliches Säureadditionssalz überführt.

6. Gegen bakterielle Infektionen wirksame pharmazeutische Zubereitungen, gekennzeichnet durch einen Gehalt an Cephemcarbonsäureestern der allgemeinen Formel I.

7. Verfahren zur Herstellung von gegen bakterielle Infektionen wirksamen pharmazeutischen Zubereitungen, dadurch gekennzeichnet, daß ein Cephemcarbonsäureester der allgemeinen Formel I mit pharmazeutisch üblichen Trägerstoffen oder Verdünnungsmitteln in eine pharmazeutisch geeignete Verabreichungsform gebracht wird.

8. Verwendung von Cephemsäureestern der allgemeinen Formel I zur Bekämpfung bakterieller Infektionen.

Patentansprüche für folgende Vertragsstaaten: ES, ER

1. Verfahren zur Herstellung von Cephemcarbonsäureestern der allgemeinen Formel I und deren physiologisch verträglichen Säureadditionssalzen,

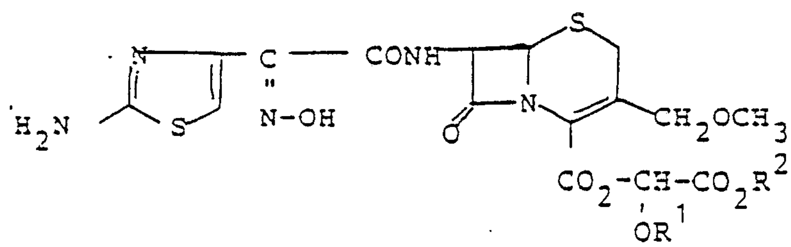

(I)

in der $R^1$ und $R^2$ $C_1$-$C_6$-Alkyl bedeuten und die HO-Gruppe in syn-Position steht, dadurch gekennzeichnet, daß man eine Verbindung der Formel II

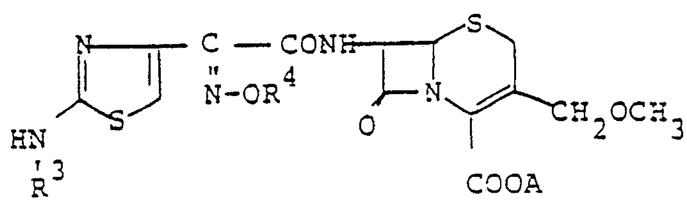

(II)

in der $R^3$ für eine Aminoschutzgruppe, $R^4$ eine leicht abspaltbare Gruppe und A ein Kation steht, mit einer Verbindung der allgemeinen Formel III

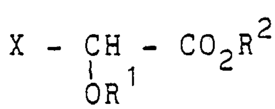

(III)

worin $R^1$ und $R^2$ die obige Bedeutung besitzen und X für eine Abgangsgruppe steht, zu dem Ester der allgemeinen Formel IV umsetzt

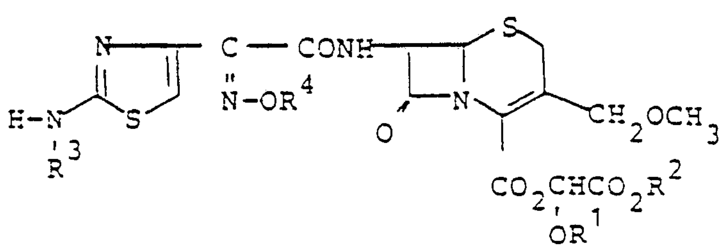

(IV)

und die Gruppen $R^3$ und $R^4$ in an sich bekannter Weise entfernt oder eine Verbindung der allgemeinen Formel V

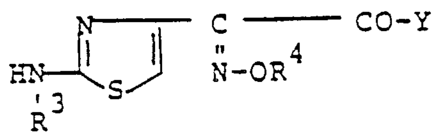

(V)

in der $R^3$ und $R^4$ die vorstehende Bedeutung besitzen und Y für eine aktivierende Gruppe steht, mit einer Verbindung der allgemeinen Formel VI

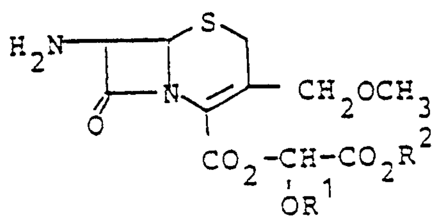

(VI)

in der $R^1$ und $R^2$ die vorstehende Bedeutung besitzen, oder mit einem Salz dieser Verbindung, zu einer Verbindung der allgemeinen Formel IV umsetzt und die Gruppen $R^3$ und $R^4$ in an sich bekannter Weise abspaltet, oder

eine Verbindung der allgemeinen Formel VII

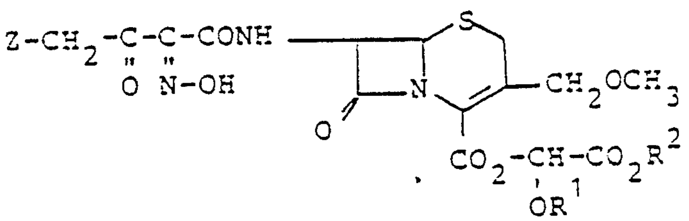

(VII)

in der Z für Halogen steht und $R^1$ und $R^2$ die vorstehende Bedeutung besitzen, mit Thioharnstoff zu Verbindungen der Formel I umsetzt und

- falls erwünscht - die erhaltenen Verbindungen in ein physiologisch verträgliches Säureadditionssalz überführt.

2. Gegen bakterielle Infektionen wirksame pharmazeutische Zubereitungen, gekennzeichnet durch einen Gehalt an Cephemcarbonsäureestern der allgemeinen Formel I.

3. Verfahren zur Herstellung von gegen bakterielle Infektionen wirksamen pharmazeutischen Zubereitungen, dadurch gekennzeichnet, daß ein Cephemcarbonsäureester der allgemeinen Formel I mit pharmazeutisch üblichen Trägerstoffen oder Verdünnungsmitteln in eine pharmazeutisch geeignete Verabreichungsform gebracht wird.

4. Verwendung von Cephemsäureestern der allgemeinen Formel I zur Bekämpfung bakterieller Infektionen.